# EUROPEAN PATENT APPLICATION

(11) **EP 2 589 378 A1**
(43) Date of publication of application: **08.05.2013**
(21) Application number: 13151617.1
(22) Date of filing: 14.10.2010
(51) Int. Cl.: A61K 9/20, A61K 31/215, A61K 9/16

(54) **Coating of cetyl myristate and/or cetyl palmitate particles**

(62) Divisional of application: 10013658.9
(71) Applicant: Deva Holding Anonim Sirketi, 34303 Istanbul (TR)
(72) Inventor: Haas, Philipp Daniel, 34303 ISTANBUL (TR); Firat, Omer Faruk, 34303 Istanbul (TR); Kandemir, Levent, 34303 ISTANBUL (TR); Ozbek, Mahmut, 34303 ISTANBUL (TR); Koc, Fikret, 34303 ISTANBUL (TR)

(57) **Abstract**

This invention is related to coated particles of cetylated fatty acids, especially cetyl myristate or cetyl palmitate or combination of cetyl myristate and cetyl palmitate by using wet granulation techniques.

## Description

### Technical Field

This invention is related to coated particles of cetyl myristate or cetyl palmitate or combination of cetyl myristate and cetyl palmitate by using wet granulation techniques. In this invention, cetyl myristate or cetyl palmitate or combination of cetyl myristate and cetyl palmitate are used as active pharmaceutical ingredients or dietary supplement.

### Background Art

Cetyl palmitate is derived from the fatty acid, palmitic acid which occurs as the glycerol ester in many oils and fats such as palm oil or Chinese vegetable tallow. A synthetic method of preparation is to react palmitoyl chloride and cetyl alcohol in the presence of magnesium. See the Merck Index, 12th edition at page 336. Reference is also made to US US3169099 A (SOCONY MOBIL OIL CO INC) 02.09.1965 patent which discloses a biosynthetic method of producing cetyl palmitate.

US 4,113,881 A (DIEHL HARRY WELDON) 12.09.1978 discloses that the administration of an effective amount of cetyl myristoleate to a mammal is useful in inhibiting or relieving the symptoms of inflammatory rheumatoid arthritis in mammals.

US 5569676 A (DIEHL, HARRY W) 29.10.1996 US 5,569,676 discloses the use of cetyl myristoleate in the treatment of osteo-arthritis.

WO 01/85162 A (MERACOL CORP LTD ET.AL.) 15.11.2001 discloses the use of cetyl myristate and/or cetyl palmitate in the treatment of irritable bowel syndrome or disease. Patent embraces that cetyl myristate comprises 50-98 wt.% of the mixture, preferably, the myristate and palmitate are in a weight ratio of 95:5. The oral dosage unit is a capsule and contains 5-400 mg of the cetyl myristate or the mixture of the cetyl myristate and the cetyl palmitate. It also includes an excipient and/or diluent, preferably silicon dioxide, calcium phosphate and/or magnesium oxide. Preferably said mixture is in a capsule and it includes a pharmaceutically acceptable excipient and/or diluents. Preferably the dosage unit includes silicon dioxide, calcium phosphate and/or magnesium oxide. Liquid formulation, where an amount of liquid equivalent to at least 4 capsules is prescribed which is to be taken 3 times daily. That is 4200 mg of cetyl myristate or the mixture of cetyl myristate and cetyl palmitate.That mixture comprises by weight 95% cetyl myristate and 5% cetyl palmitate by weight In addition added excipients were present in the non gelatin two part capsule case.

WO 01/85163 A (MERACOL CORP LTD ET.AL.) 15.11.2001 discloses the use of cetyl myristate and/or cetyl palmitate in the treatment of eczema and/or psoriasis. Accordingly, capsule also includes a pharmaceutically acceptable excipient and/or diluent. These are silicon dioxide, calcium phosphate and/or magnesium oxide. The dosage unit can also be a wax-like solid or can be an orally consumable liquid composition (eg; made up with a general pharmacy type carrier such as methyl cellulose).

WO 2005/118070 A (MERACOL CORP LTD ET.AL.) 15.12.2005 discloses the treatment of multiple sclerosis with the use of cetyl myristate and/or cetyl palmitate.The cetyl myristate; or combination of cetyl myristate and cetyl palmitate is administered simultaneously, separately or sequentially.

WO 03/018731 A (MERACOL CORP LTD) 06.03.2003 defines the process prepares a mixture of cetyl myristate (50-98 wt.%) and cetyl palmitate, for use in the formulation of cosmetics and pharmaceuticals.

WO 03/045374 A (MERACOL CORP LTD ET.AL.) 05.06.2003 discloses the use of cetyl myristate and/or cetyl palmitate in a method of treatment and/or prophylaxis of a mammal for at least the symptoms of treating asthma, chronic obstruc tive pulmonary disease and/or other respiratory difficulties.

WO 03/026640 A (MERACOL CORP LTD ET.AL.) 03.04.2003 discloses the use of cetyl myristate and/or cetyl palmitate in the treatment of food allergies and/or food intolerances.

WO 01/85164 A (MERACOL CORP LTD ET.AL.) 15.11.2001 discloses the use of cetyl myristate and/or cetyl palmitate in the treatment of herpes.

### Summary of invention

This invention discloses that particles of cetylated fatty acids, especially cetyl myristate or cetyl palmitate or combination of cetyl palmitate and cetyl myristate are coated. Present invention also embraces wet granulation method of cetyl myristate or cetyl palmitate or combination of cetyl palmitate and cetyl myristate.

### Technical Problem

Cetyl myristate or cetyl palmitate or combination of cetyl myristate and cetyl palmitate can be used as active pharmaceutical ingredients (API) in pharmaceutical formulations in addition to excipient properties. However both cetyl myristate and cetyl palmitate are waxy ingredients and thus in formulation stage, they are adhered onto the surfaces of formulation equipments therefore it is hard to granulate such API's in processes. Thus such processes that non-adhering and eligible granulation methods are needed.

### Solution to Problem

It is invented that sticking problem is solved by coating of particles of cetyl myristate and cetyl palmitate by using wet granulation techniques. Coating provides a minimisation and isolation of the waxy surface of particles. In terms of solution,it is also delineated that percentages of API's and excipients,weight ratios,useful solvents and temporary conditions.

### Description of embodiments

Cetyl myristate and cetyl palmitate are sticky and tend to adhere to surfaces of granulation and formulation equipments .

This invention discloses processability of cetyl myristate and cetyl palmitate and also the ability not to stick or adhere to equipments.

This invention also embraces to provide a wet granulation and/or coating method free from adhesion .

According to this invention, it is invented that adhering problems in Cetyl myristate and cetyl palmitate formulations are eliminated by coating of particles of active ingredients. Coating can be carried out by fluid bed,high or low shear mixer,etc.

In one aspect of this invention particles of cetyl myristate and cetyl palmitate are coated in unison. Accordingly particles of cetyl myristate and cetyl palmitate can be directly coated or can be coated after admixturing with pharmaceutical excipient or mixtures of pharmaceutical excipients .

In another aspect of this invention,cetyl myristate and cetyl palmitate particles can be coated after treatment with a pharmaceutical excipient or mixtures of pharmaceutical excipients.

In a further aspect of this invention cetyl myristate and cetyl palmitate particles can be treated with a pharmaceutical excipient or mixtures of pharmaceutical excipients in the course of coating.

Yet another aspect of this invention, cetyl myristate and cetyl palmitate granules can be coated. Granulation can be made through using of wet granulation, dry granulation and other suitable granulation methods. Wet granulation is preferred.

Coating can be performed by known methods of pharmaceutical technology such as fluid bed, high shear mixer,low sheer mixer etc.

In coating, wet granulation method is preferred. However in wet granulation it is required that a technical problem should be solved. Said problem arises from using of water in wet granulation. When water is used alone as a solvent,huge parts of cetyl myristate and cetyl palmitate start to gel and thus preparing of pharmaceutical formulation becomes nearly impossible. To solve said technical problem it is invented that in wet granulation phase solvent or mixtures of solvents are used other than solely water. Water can be used as a mixed manner with a solvent or mixtures of solvents but according to this invention it *per se* cannot be used in wet granulation.

Wet granulation method includes following intragranulation and extragranulation steps : **I.Intragranulation** : a. a binder ,a disintegrant, cetyl myristate, cetyl palmitate are mixed, b.a binder and plasticizer is dissolved in a solvent other than solely water, c. materials obtained from step a and step b are mixed in a mixer,preferably high shear mixer, **II.Extragranulation**: a.obtained granules from intragranulation step are dried, b. disintegrant and lubricant are mixed, c. obtained materials from step a and step b are mixed in a mixer.

In combination of cetyl myristate and cetyl palmitate, granules of each active pharmaceutical ingredients can be seperately granulated and then assemblied or both active pharmaceutical ingredient can be granulated together or one active pharmaceutical ingredient is granulated and then it is assemblied with other non-granulated active pharmaceutical ingredient. It is mostly preferred that both active pharmaceutical ingredients are granulated together.

Final materials can be filed into capsules or can be pressed as tablet or can be another suitable pharmaceutical form.

Intragranulation phase comprises filler is from about 0,5 % to about 10 %, disintegrant is from about 1 % to about 10 %, cetyl myrisate as active pharmaceutical ingredient is from about 40 % to about 99,5 %, cetyl palmitate as active pharmaceutical ingredient is from about 0,5 % to about 10 %, plasticizer is from about 0,25 % to about 6 %, binder is from about 1 % to about 6 % by weight of total intragranulation phase.

Intragranulation phase preferably comprises filler is 3,76 % , disintegrant is 5,00 %, cetyl myrisate as active pharmaceutical ingredient is 83,32 % ,cetyl palmitate as active pharmaceutical ingredient is 4,17 %, plasticizer is 1,25 %, binder is 2,50 % by weight of total intragranulation phase.

Most preferably, intragranulation phase comprises filler is 15 mg , disintegrant is 20 mg, cetyl myrisate as active pharmaceutical ingredient is 333,3 mg, cetyl palmitate as active pharmaceutical ingredient is 16,7 mg, plasticizer is 5 mg, binder is 10 mg.

Extragranulation comprises intragranular material obtained from intragranulation phase, lubricant and disintegrant.

Extragranulation phase comprises intragranular material is from about 40 % to 99,5, lubricant is from about 0,5 % to 10 %, disintegrant is from about 0,5 % to 10 % by weight of total extragranulation phase.

Extragranulation phase preferably comprises intragranular material is 95,2 %, lubricant is 2,3 %, disintegrant is 2,3 % by weight of total extragranulation phase.

Most preferably,extragranulation phase comprises intragranular material is 400 mg, lubricant is 10 mg and disintegrant is 10 mg.

Pharmaceutical composition comprising extragranulation and intragranulation in a weight ratio of intragranulation phase to extragranulation phase is from 5:1 to 1:5. Preferably weight ratio is 1:1,05

According to this invention pharmaceutical composition comprises diluent/filler is from 0.5% to 20%, disintegrant is from 1% to 15%, cetyl myrisate as active pharmaceutical ingredient is from 40% to 99.5%,cetyl palmitate as active pharmaceutical ingredient is from 0.5% to 30%,plasticizer is from 0.1% to 10%, binder is from 0.1% to 20%, lubricant is from 0.1 % to 20 %, by weight of total pharmaceutical composition. Preferebly,pharmaceutical composition comprises diluents/filler is 3.5%, disintegrant is 7.2%, cetyl myrisate as active pharmaceutical ingredient is 79.3%, cetyl palmitate as active pharmaceutical ingredient is 3.9%,plasticizer is 1.2%, binder is 2.5% , lubricant is 2.4%, by weight of total pharmaceutical composition. If pharmaceutical composition is filled into a capsule, capsule weight is not included to "total pharmaceutical composition".

In accordance with present invention, the pharmaceutical composition of cetyl myristate and cetyl palmitate combination comprises disintegrant, lubricant, binder,diluent/filler,coating agents and the like.

According to this invention the pharmaceutical composition of cetyl myristate or cetyl palmitate or combination of cetyl myristate and cetyl palmitate comprising multistage disintegrant accompanying wherein disintegrant or mixture of disintegrants are separated into two parts wherein initial part of disintegrant or mixture of disintegrants are used in first phase and residual portion of said disintegrant or mixture of disintegrants are used in additional phase or additional phases. Herein,first phase is preferably intragranular phase and additional phase is preferably extragranular phase. Multistage disintegrant accompanying can be performed without separation of a disintegrant mass.

Weight ratio of extragranular disintegrant and intragranular disintegrant is from 5:1 to 1:5. Preferably weight ratio is 1:2 (extragranular disintegrant : intragranular disintegrant).

In another aspect, it is invented that pharmaceutical composition of cetyl myristate or cetyl palmitate or combination of cetyl myristate and cetyl palmitate should be granulated and granules are dried under 45°C. Exceeding 45°C, active ingredient becomes a coagulate and smear form and granulation is impossible. For instance granules are preferably dried maximum 40°C.

In yet another aspect of this invention is to provide a specific proportional weight ratio of excipients to active pharmaceutical ingredients. If a weight ratio is not taken into account,total weight of pharmaceutical composition wold be so heavy and huge volume for ingestion of patients. To solve this technical problem, it is invented that weight ratio of excipients to active pharmaceutical ingredients should not exceed 1:30 in intragranulation phase. Preferably weight ratio of excipients to active pharmaceutical ingredients is 1:7 in intragranulation phase. In extragranular phase, weight ratio of excipients to active pharmaceutical ingredients should not exceed 1:50. Preferably weight ratio of excipients to active pharmaceutical ingredients is 1:17,5 in extragranulation phase.

Disintegrants are, but not limited to, alginic acid, chitosan, pectins, cation exchange resins, magnesium silicates, aluminium silicates,mixtures thereof and the like.

Lubricants/Glidants are, but not limited to, magnesium stearate,calcium stearate,hydrogenated castor oil,glyceryl behenate,glyceryl monostearate,glyceryl palmitostearate,leucine,mineral oil, light mineral oil, myristic acid,palmitic acid,polyethylene glycol,potassium benzoate,sodium benzoate,sodium lauryl sulfate,sodium stearyl fumarate,stearic acid,talc, hydrogenated vegetable oil,zinc stearate, magnesium lauryl sulphate,sodium stearyl fumarate, polyethylene glycol, stearic acid, colloidal silicon dioxide or mixtures thereof and the like. Preferred lubricant is magnesium stearate. Two different kinds of lubricants are preferred to be benefited from different proporties of lubrication.

Binders are, but not limited to, sodium alginate, cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pre-gelatinized starch, sugars, trehalose, glucose,tragacanth, sorbitol, acacia, alginates, carrageenan, xanthan gum, locust bean gum and gum arabic, waxes,polyacrylamide, mixtures thereof, and the like.

Diluents/fillers are, but not limited to, mannitol, sorbitol, xylitol, microcrystalline cellulose, silicified microcrystalline cellulose , hydroxypropyl methylcellulose, hydroxypropyl cellulose , pullulan and fast dissolving carbohydrates such as Pharmaburst^{™}, mixtures thereof and the like.

Plasticizers are , but not limited to, polyethylene glycol, propylene glycol, triacetin, dimethyl phthalate, diethyl phthalate, dibutyl phthalate, dibutyl sebacate, triethyl citrate, tributyl citrate, triethyl acetyl citrate, castor oil , acetylated monoglycerides, mixtures thereof and the like. Plasticizer is used to have hard granulate.

### Example

**Table 1**

| **INGREDIENTS/FUNCTIONS** | **mg** |
|---|---|
| **INTRAGRANULAR PHASE** | |
| Cetyl Myristate/API | 333.3 |
| Cetyl Palmitate/API | 16.7 |
| Disintegrant | 20.0 |
| Diluent/Filler | 15.0 |
| Binder | 10.0 |
| Plasticizer | 5.0 |
| Ethanol (Evapourated,not calculated in final granules) | 120 |
| **TOTAL (INTRAGRANULAR PHASE)** | **400** |

| **EXTRAGRANULAR PHASE** | |
|---|---|
| Lubricant | 10 |
| Disintegrant | 10 |
| **TOTAL (EXTRAGRANULAR PHASE)** | **20** |
| **TOTAL(EXTRAGRANULAR+INTRAG RANULAR)** | **420.0** |

## Claims

1. A pharmaceutical composition of cetyl myristate or cetyl palmitate or combination of cetyl myristate and cetyl palmitate **characterized in that** granulation is prepared and/or dried under 45°C.
